# EUROPEAN PATENT SPECIFICATION

(11) **EP 4 591 917 B1**
(45) Date of publication and mention of the grant of the patent: **26.08.2026**
(21) Application number: 25180631.1
(22) Date of filing: 04.10.2018
(51) Int. Cl.: A61M 25/00, A61M 27/00

(54) **SYSTEMS FOR TREATING ALONG THE CENTRAL NERVOUS SYSTEM**
SYSTEME ZUR BEHANDLUNG ENTLANG DES ZENTRALEN NERVENSYSTEMS
SYSTÈMES DE TRAITEMENT LE LONG DU SYSTÈME NERVEUX CENTRAL

(30) Priority: 05.10.2017 US 201762568412 P; 14.12.2017 US 201762598846 P; 14.03.2018 US 201862642873 P; 18.06.2018 US 201862686413 P
(43) Date of publication of application: 30.07.2025
(62) Document number(s) of the earlier application(s) in accordance with Art. 76 EPC: 18792799.1 / 3 691 730
(73) Proprietor: Pharaoh Neuro, Inc., Minneapolis, MN 55405 (US)
(72) Inventor: LAD, Shivanand P., Durham, North Carolina, 27713 (US); MCCABE, Aaron R., Edina, Minnesota, 55424 (US); ZITELLA VERBICK, Laura Marie, North St. Paul, Minnesota, 55109 (US); HEDSTROM, Blake, Minneapolis, Minnesota, 55407 (US); STOLL, Matthew, Minneapolis, Minnesota, 55419 (US); MONDRY, Jack, Edina, Minnesota, 55435 (US)
(74) Representative: Vossius & Partner Patentanwälte Rechtsanwälte mbB

(56) References cited:
- WO-A1-2011/114260
- WO-A1-2017/023419
- US-A- 4 904 237
- US-A1- 2008 249 501
- US-A1- 2010 305 492

## Description

### Cross-Reference to Related Applications

This application claims priority under 35 U.S.C. §119 to U.S. Provisional Application Serial No. 62/568,412, filed October 5, 2017. This application also claims priority under 35 U.S.C. §119 to U.S. Provisional Application Serial No. 62/598,846, filed December 14, 2017. This application also claims priority under 35 U.S.C. §119 to U.S. Provisional Application Serial No. 62/642,873, filed March 14, 2018. This application also claims priority under 35 U.S.C. §119 to U.S. Provisional Application Serial No. 62/686,413, filed June 18, 2018.

### Technical Field

The present disclosure relates to systems, catheters, and methods for treating along the central nervous system.

### Background

A wide variety of medical devices have been developed for medical use. Some of these devices include guidewires, catheters, and the like. These devices are manufactured by any one of a variety of different manufacturing methods and may be used according to any one of a variety of methods. Of the known medical devices and methods, each has certain advantages and disadvantages. There is an ongoing need to provide alternative medical devices as well as alternative methods for manufacturing and using medical devices.

US 2010/0305492 A1 relates to methods and systems for conditioning cerebrospinal fluid (CSF). The methods provide for efficiently removing target compounds from CSF. The systems provide for a multilumen flow path and exchange of a majority volume portion of CSF in the CSF space. The removal and/or delivery of specific compounds can be tailored to the pathology of the specific disease. The removal is targeted and specific, for example, through the use of specific size-exclusion thresholds, antibodies against specific toxins, and other chromatographic techniques, as well as delivery and/or removal of targeted therapeutic agents.

WO 2017/023419 A1 relates to a system and a method for filtering materials from biologic fluids. The system may be used to filter cerebrospinal fluid from a human or animal subject. The method may include the steps of withdrawing fluid comprising CSF, filtering the volume into permeate and retentate by passing the fluid through a tangential flow filter, and returning the permeate to the subject. During operation of the system, various parameters may be modified, such as flow rate.

WO 2011/114260 A1 relates to systems and methods for the removal of toxins from the cerebrospinal fluid (CSF) are disclosed. A system comprises a first catheter that can be inserted into a brain ventricle or into the cervical spine, and a second catheter that can be inserted into the lumbar spine.

US 4 904 237 A relates to an apparatus and method for exchanging cerebrospinal fluid. The apparatus withdraws cerebrospinal fluid from the lumbar subarachnoid space, filters, cools, pH adjusts and performs diagnostic/monitoring functions on the fluid. It then returns purified and/or artificial spinal fluid to the subarachnoid space. The apparatus monitors fluid temperature and core body temperature. Furthermore, it detects increased subarachnoid hemorrhage or repeat subarachnoid hemorrhage.

US 2008/249501 A1 relates to methods for simultaneous injection and aspiration of fluids during a medical procedure, e. g. to methods for operating medical devices within the subarachnoid space of the spinal column to gain access to the ventricles of the brain, as well as the surrounding cranial subarachnoid space.

### Summary

The invention is defined in the appended claims. Aspects, embodiments and examples disclosed herein which do not fall within the scope of the appended claims do not form part of the invention and are merely provided for illustrative purposes.

This disclosure provides design, material, manufacturing method, and use alternatives for medical devices. An example method for treating cancer is disclosed. The method comprises: removing cerebrospinal fluid from a patient with cancer; filtering the cerebrospinal fluid with a filtration system to remove cancer cells; and returning the filtered cerebrospinal fluid to the patient.

Alternatively or additionally to any of the embodiments above, removing cerebrospinal fluid from a patient with cancer includes removing cerebrospinal fluid from a lumbar cerebrospinal space of the patient.

Alternatively or additionally to any of the embodiments above, removing cerebrospinal fluid from a patient with cancer includes disposing a catheter system within a cerebrospinal space.

Alternatively or additionally to any of the embodiments above, the catheter system includes a catheter shaft having an inner shaft and an outer shaft.

Alternatively or additionally to any of the embodiments above, the inner shaft has a plurality of openings formed therein.

Alternatively or additionally to any of the embodiments above, the outer shaft has a plurality of openings formed therein.

Alternatively or additionally to any of the embodiments above, the inner shaft has a first plurality of openings formed therein, wherein the outer shaft has a second plurality of openings formed therein, and wherein at least some of the first plurality of openings differ in size, shape, or both from at least some of the second plurality of openings.

Alternatively or additionally to any of the embodiments above, the inner shaft defines an infusion lumen.

Alternatively or additionally to any of the embodiments above, returning the filtered cerebrospinal fluid to the patient includes returning the filtered cerebrospinal fluid through the infusion lumen.

Alternatively or additionally to any of the embodiments above, further comprising infusing a chemotherapy agent through the infusion lumen.

Alternatively or additionally to any of the embodiments above, an aspiration lumen is defined between the inner shaft and the outer shaft.

Alternatively or additionally to any of the embodiments above, removing cerebrospinal fluid from a patient with cancer includes removing cerebrospinal fluid through the aspiration lumen.

Alternatively or additionally to any of the embodiments above, removing cerebrospinal fluid from a patient with cancer includes removing tumor cells.

Altematively or additionally to any of the embodiments above, further comprising infusing a chemotherapy agent into the patient.

Alternatively or additionally to any of the embodiments above, the chemotherapy agent includes methotrexate.

Alternatively or additionally to any of the embodiments above, infusing a chemotherapy agent into the patient includes implanting an Ommaya reservoir and infusing the chemotherapy agent into the patient via the Ommaya reservoir.

Alternatively or additionally to any of the embodiments above, infusing a chemotherapy agent into the patient includes infusing the chemotherapy agent into the patient with a catheter system.

Alternatively or additionally to any of the embodiments above, infusing the chemotherapy agent into the patient with a catheter system includes disposing the catheter system within a cerebrospinal space.

Alternatively or additionally to any of the embodiments above, disposing the catheter system within a cerebrospinal space includes disposing the catheter system a lumbar cerebrospinal fluid space.

Alternatively or additionally to any of the embodiments above, the filtration system includes a 5kDa tangential flow filter, a 100 kDa tangential flow filter, or both.

Alternatively or additionally to any of the embodiments above, further comprising removing cerebrospinal fluid from a cranial space.

Alternatively or additionally to any of the embodiments above, further comprising removing cerebrospinal fluid from a cranial space with a catheter.

Alternatively or additionally to any of the embodiments above, removing cerebrospinal fluid from a patient with cancer includes removing cerebrospinal fluid from a first location with a first catheter.

Alternatively or additionally to any of the embodiments above, returning the filtered cerebrospinal fluid to the patient includes returning the filtered cerebrospinal fluid to a first region adjacent to the first location with the first catheter.

Alternatively or additionally to any of the embodiments above, further comprising removing cerebrospinal fluid from a second location with a second catheter.

Alternatively or additionally to any of the embodiments above, further comprising infusing filtered cerebrospinal fluid to a second region adjacent to the second location with the second catheter.

A method for treating cancer is disclosed. The method comprises: disposing a catheter in a cerebrospinal space of a patient with cancer; aspirating cerebrospinal fluid from the patient with the catheter; filtering the cerebrospinal fluid with a filtration system to remove cancer cells; and returning the filtered cerebrospinal fluid to the patient with the catheter.

Alternatively or additionally to any of the embodiments above, aspirating cerebrospinal fluid from the patient with the catheter includes aspirating cerebrospinal fluid from a lumbar cerebrospinal space of the patient.

Alternatively or additionally to any of the embodiments above, the catheter includes a catheter shaft having an inner shaft and an outer shaft.

Alternatively or additionally to any of the embodiments above, the inner shaft has a plurality of openings formed therein.

Alternatively or additionally to any of the embodiments above, the outer shaft has a plurality of openings formed therein.

Alternatively or additionally to any of the embodiments above, the inner shaft has a first plurality of openings formed therein, wherein the outer shaft has a second plurality of openings formed therein, and wherein at least some of the first plurality of openings differ in size, shape, or both from at least some of the second plurality of openings.

Alternatively or additionally to any of the embodiments above, further comprising infusing a chemotherapy agent into the patient.

Alternatively or additionally to any of the embodiments above, the chemotherapy agent includes methotrexate.

Alternatively or additionally to any of the embodiments above, infusing a chemotherapy agent into the patient includes infusing the chemotherapy agent into the patient with an Ommaya reservoir.

Alternatively or additionally to any of the embodiments above, infusing a chemotherapy agent into the patient includes infusing the chemotherapy agent into the patient with the catheter.

Alternatively or additionally to any of the embodiments above, the filtration system includes a 5kDa tangential flow filter, a 100 kDa tangential flow filter, or both.

A method for treating cancer is disclosed. The method comprises: disposing a catheter in a cerebrospinal space of a patient with cancer; aspirating cerebrospinal fluid from the patient with the catheter; conditioning the cerebrospinal fluid with a filtration system; returning the filtered cerebrospinal fluid to the patient with the catheter; and infusing a chemotherapy agent into the cerebrospinal space with the catheter.

Alternatively or additionally to any of the embodiments above, aspirating cerebrospinal fluid from the patient with the catheter includes aspirating cerebrospinal fluid from a lumbar cerebrospinal space of the patient.

Alternatively or additionally to any of the embodiments above, the catheter includes a catheter shaft having an inner shaft and an outer shaft.

Alternatively or additionally to any of the embodiments above, the inner shaft has a plurality of openings formed therein.

Alternatively or additionally to any of the embodiments above, the outer shaft has a plurality of openings formed therein.

Alternatively or additionally to any of the embodiments above, the inner shaft has a first plurality of openings formed therein, wherein the outer shaft has a second plurality of openings formed therein, and wherein at least some of the first plurality of openings differ in size, shape, or both from at least some of the second plurality of openings.

Alternatively or additionally to any of the embodiments above, further comprising infusing a second chemotherapy agent into the patient with an Ommaya reservoir.

Alternatively or additionally to any of the embodiments above, the chemotherapy agent and the second chemotherapy agent are the same.

Alternatively or additionally to any of the embodiments above, the chemotherapy agent, the second chemotherapy agent, or both include methotrexate.

Alternatively or additionally to any of the embodiments above, the filtration system includes a 5kDa tangential flow filter, a 100 kDa tangential flow filter, or both.

A system for treating cancer is disclosed. The system comprises: a catheter including an inner shaft and an outer shaft; wherein an infusion lumen is defined within the inner shaft; wherein an aspiration lumen is defined between the inner shaft and the outer shaft; a first plurality of openings in the inner shaft, the first plurality of openings being in fluid communication with the infusion lumen; a second plurality of openings in the outer shaft, the second plurality of openings being in fluid communication with the aspiration lumen; a filtration system in fluid communication with the infusion lumen and the aspiration lumen, the filtration system including one or more filters designed to filter cancer cells from cerebrospinal fluid.

Alternatively or additionally to any of the embodiments above, at least some of the first plurality of openings differ in size, shape, or both from at least some of the second plurality of openings.

Alternatively or additionally to any of the embodiments above, the filtration system includes a 5kDa tangential flow filter, a 100 kDa tangential flow filter, or both.

A method for treating amyotrophic lateral sclerosis (ALS) is disclosed. The method comprises: removing cerebrospinal fluid from a patient with ALS; filtering the cerebrospinal fluid with a filtration system to remove oxidative and/or inflammatory agents; and returning the filtered cerebrospinal fluid to the patient.

Alternatively or additionally to any of the embodiments above, removing cerebrospinal fluid from a patient with ALS includes removing cerebrospinal fluid from a lumbar cerebrospinal space of the patient.

Alternatively or additionally to any of the embodiments above, removing cerebrospinal fluid from a patient with ALS includes disposing a catheter system within a cerebrospinal space.

Alternatively or additionally to any of the embodiments above, the catheter system includes a catheter shaft having an inner shaft and an outer shaft.

Alternatively or additionally to any of the embodiments above, the inner shaft has a plurality of openings formed therein.

Alternatively or additionally to any of the embodiments above, the outer shaft has a plurality of openings formed therein.

Alternatively or additionally to any of the embodiments above, the inner shaft has a first plurality of openings formed therein, wherein the outer shaft has a second plurality of openings formed therein, and wherein at least some of the first plurality of openings differ in size, shape, or both from at least some of the second plurality of openings.

Alternatively or additionally to any of the embodiments above, the inner shaft defines an infusion lumen.

Alternatively or additionally to any of the embodiments above, returning the filtered cerebrospinal fluid to the patient includes returning the filtered cerebrospinal fluid through the infusion lumen.

Alternatively or additionally to any of the embodiments above, further comprising infusing a drug through the infusion lumen.

Alternatively or additionally to any of the embodiments above, an aspiration lumen is defined between the inner shaft and the outer shaft.

Alternatively or additionally to any of the embodiments above, removing cerebrospinal fluid from a patient with ALS includes removing cerebrospinal fluid through the aspiration lumen.

Alternatively or additionally to any of the embodiments above, removing cerebrospinal fluid from a patient with cancer includes removing one or more of insoluble superoxide dismutase-1 (SOD1), glutamate, neurofilament protein, and anti-GM1 ganglioside antibodies.

Alternatively or additionally to any of the embodiments above, wherein the filtration system includes a 5kDa tangential flow filter, a 100 kDa tangential flow filter, or both.

Alternatively or additionally to any of the embodiments above, wherein the filtration system includes an electrofilter.

A method for treating herpes simplex encephalitis (HSE) is disclosed. The method comprises: removing cerebrospinal fluid from a patient with HSE; filtering the cerebrospinal fluid with a filtration system to remove oxidative and/or inflammatory agents; and returning the filtered cerebrospinal fluid to the patient.

Alternatively or additionally to any of the embodiments above, removing cerebrospinal fluid from a patient with HSE includes removing cerebrospinal fluid from a lumbar cerebrospinal space of the patient.

Alternatively or additionally to any of the embodiments above, removing cerebrospinal fluid from a patient with HSE includes disposing a catheter system within a cerebrospinal space.

Alternatively or additionally to any of the embodiments above, the catheter system includes a catheter shaft having an inner shaft and an outer shaft.

Alternatively or additionally to any of the embodiments above, the inner shaft has a plurality of openings formed therein.

Alternatively or additionally to any of the embodiments above, the outer shaft has a plurality of openings formed therein.

Alternatively or additionally to any of the embodiments above, the inner shaft has a first plurality of openings formed therein, wherein the outer shaft has a second plurality of openings formed therein, and wherein at least some of the first plurality of openings differ in size, shape, or both from at least some of the second plurality of openings.

Alternatively or additionally to any of the embodiments above, the inner shaft defines an infusion lumen.

Alternatively or additionally to any of the embodiments above, returning the filtered cerebrospinal fluid to the patient includes returning the filtered cerebrospinal fluid through the infusion lumen.

Alternatively or additionally to any of the embodiments above, further comprising infusing a drug through the infusion lumen.

Alternatively or additionally to any of the embodiments above, an aspiration lumen is defined between the inner shaft and the outer shaft.

Alternatively or additionally to any of the embodiments above, removing cerebrospinal fluid from a patient with HSE includes removing cerebrospinal fluid through the aspiration lumen.

Alternatively or additionally to any of the embodiments above, the filtration system includes a 5kDa tangential flow filter, a 100 kDa tangential flow filter, or both.

Alternatively or additionally to any of the embodiments above, the filtration system includes an electrofilter.

A method for treating multiple sclerosis (MS) is disclosed. The method comprises: removing cerebrospinal fluid from a patient with MS; filtering the cerebrospinal fluid with a filtration system to remove oxidative and/or inflammatory agents; and returning the filtered cerebrospinal fluid to the patient.

Alternatively or additionally to any of the embodiments above, removing cerebrospinal fluid from a patient with MS includes removing cerebrospinal fluid from a lumbar cerebrospinal space of the patient.

Alternatively or additionally to any of the embodiments above, removing cerebrospinal fluid from a patient with MS includes disposing a catheter system within a cerebrospinal space.

Alternatively or additionally to any of the embodiments above, the catheter system includes a catheter shaft having an inner shaft and an outer shaft.

Alternatively or additionally to any of the embodiments above, the inner shaft has a plurality of openings formed therein.

Alternatively or additionally to any of the embodiments above, the outer shaft has a plurality of openings formed therein.

Alternatively or additionally to any of the embodiments above, the inner shaft has a first plurality of openings formed therein, wherein the outer shaft has a second plurality of openings formed therein, and wherein at least some of the first plurality of openings differ in size, shape, or both from at least some of the second plurality of openings.

Alternatively or additionally to any of the embodiments above, the inner shaft defines an infusion lumen.

Alternatively or additionally to any of the embodiments above, returning the filtered cerebrospinal fluid to the patient includes returning the filtered cerebrospinal fluid through the infusion lumen.

Alternatively or additionally to any of the embodiments above, further comprising infusing a drug through the infusion lumen.

Alternatively or additionally to any of the embodiments above, an aspiration lumen is defined between the inner shaft and the outer shaft.

Alternatively or additionally to any of the embodiments above, removing cerebrospinal fluid from a patient with MS includes removing cerebrospinal fluid through the aspiration lumen.

Alternatively or additionally to any of the embodiments above, the filtration system includes a 5kDa tangential flow filter, a 100 kDa tangential flow filter, or both.

Alternatively or additionally to any of the embodiments above, the filtration system includes an electrofilter.

A method for treating human immunodeficiency virus (HIV) is disclosed. The method comprises: removing cerebrospinal fluid from a patient with HIV; filtering the cerebrospinal fluid with a filtration system to remove viral, oxidative, and/or inflammatory agents; and returning the filtered cerebrospinal fluid to the patient.

Alternatively or additionally to any of the embodiments above, removing cerebrospinal fluid from a patient with HIV includes removing cerebrospinal fluid from a lumbar cerebrospinal space of the patient.

Alternatively or additionally to any of the embodiments above, removing cerebrospinal fluid from a patient with HIV includes disposing a catheter system within a cerebrospinal space.

Alternatively or additionally to any of the embodiments above, the catheter system includes a catheter shaft having an inner shaft and an outer shaft.

Alternatively or additionally to any of the embodiments above, the inner shaft has a plurality of openings formed therein.

Alternatively or additionally to any of the embodiments above, the outer shaft has a plurality of openings formed therein.

Alternatively or additionally to any of the embodiments above, the inner shaft has a first plurality of openings formed therein, wherein the outer shaft has a second plurality of openings formed therein, and wherein at least some of the first plurality of openings differ in size, shape, or both from at least some of the second plurality of openings.

Alternatively or additionally to any of the embodiments above, the inner shaft defines an infusion lumen.

Alternatively or additionally to any of the embodiments above, returning the filtered cerebrospinal fluid to the patient includes returning the filtered cerebrospinal fluid through the infusion lumen.

Alternatively or additionally to any of the embodiments above, further comprising infusing a drug through the infusion lumen.

Alternatively or additionally to any of the embodiments above, an aspiration lumen is defined between the inner shaft and the outer shaft.

Alternatively or additionally to any of the embodiments above, removing cerebrospinal fluid from a patient with HIV includes removing cerebrospinal fluid through the aspiration lumen.

Alternatively or additionally to any of the embodiments above, the filtration system includes a 5kDa tangential flow filter, a 100 kDa tangential flow filter, or both.

Alternatively or additionally to any of the embodiments above, the filtration system includes an electrofilter.

A method for treating Guillain-Barré syndrome (GBS) is disclosed. The method comprises: removing cerebrospinal fluid from a patient with GBS; filtering the cerebrospinal fluid with a filtration system to remove oxidative and/or inflammatory agents; and returning the filtered cerebrospinal fluid to the patient.

Alternatively or additionally to any of the embodiments above, removing cerebrospinal fluid from a patient with GBS includes removing cerebrospinal fluid from a lumbar cerebrospinal space of the patient.

Alternatively or additionally to any of the embodiments above, removing cerebrospinal fluid from a patient with GBS includes disposing a catheter system within a cerebrospinal space.

Alternatively or additionally to any of the embodiments above, the catheter system includes a catheter shaft having an inner shaft and an outer shaft.

Alternatively or additionally to any of the embodiments above, the inner shaft has a plurality of openings formed therein.

Alternatively or additionally to any of the embodiments above, the outer shaft has a plurality of openings formed therein.

Alternatively or additionally to any of the embodiments above, the inner shaft has a first plurality of openings formed therein, wherein the outer shaft has a second plurality of openings formed therein, and wherein at least some of the first plurality of openings differ in size, shape, or both from at least some of the second plurality of openings.

Alternatively or additionally to any of the embodiments above, the inner shaft defines an infusion lumen.

Alternatively or additionally to any of the embodiments above, returning the filtered cerebrospinal fluid to the patient includes returning the filtered cerebrospinal fluid through the infusion lumen.

Alternatively or additionally to any of the embodiments above, further comprising infusing a drug through the infusion lumen.

Alternatively or additionally to any of the embodiments above, an aspiration lumen is defined between the inner shaft and the outer shaft.

Alternatively or additionally to any of the embodiments above, removing cerebrospinal fluid from a patient with GBS includes removing cerebrospinal fluid through the aspiration lumen.

Alternatively or additionally to any of the embodiments above, the filtration system includes a 5kDa tangential flow filter, a 100 kDa tangential flow filter, or both.

Alternatively or additionally to any of the embodiments above, the filtration system includes an electrofilter.

A system for removing materials from cerebrospinal fluid is disclosed. The system comprisse: a first port for providing access a cerebrospinal space at a first location; a first catheter coupled to the first port, the first catheter being designed to remove cerebrospinal fluid from the first location; a filtration system coupled to the first catheter, the filtration system being designed to filter the cerebrospinal fluid removed from the first location; a second port for providing access a cerebrospinal space at a second location; and a second catheter coupled to the second port, the first catheter being designed to returned the filtered cerebrospinal fluid.

Alternatively or additionally to any of the embodiments above, the first location is a lumbar region of a patient.

Alternatively or additionally to any of the embodiments above, the second location is adjacent to a cranial region of the patient.

A method for ameliorating a symptom of bacterial meningitis in a patient is disclosed. The method comprises: selecting a patient having a symptom of bacterial meningitis; removing cerebrospinal fluid from a first location in a lumbar cerebrospinal fluid space of the patient; removing at a flow rate one or more meningitis causing bacterial pathogen from the removed cerebrospinal fluid, thereby conditioning the cerebrospinal fluid; and returning the conditioned cerebrospinal fluid to the patient at a second location in a cervical cerebrospinal fluid space, a thoracic cerebrospinal fluid space, or a ventricle of the patient; wherein the removing and returning steps are performed concurrently using one or more catheters, each catheter comprising one or more lumens.

Alternatively or additionally to any of the embodiments above, the cerebrospinal fluid is returned to the patient at substantially the same flow rate at which it is removed.

Alternatively or additionally to any of the embodiments above, further including removing at the flow rate one or more endotoxins from the removed cerebrospinal fluid.

Alternatively or additionally to any of the embodiments above, further including removing at the flow rate one or more cytokines from the removed cerebrospinal fluid.

Alternatively or additionally to any of the embodiments above, the removing of the one or more meningitis causing bacterial pathogens is achieved using a filter system particularly configured to remove the one or more meningitis causing bacterial pathogens.

Alternatively or additionally to any of the embodiments above, the removing of the one or more endotoxins is achieved using a filter system particularly configured to remove the one or more endotoxins.

Alternatively or additionally to any of the embodiments above, the removing of the one or more cytokines is achieved using a filter system particularly configured to remove the one or more cytokines.

Alternatively or additionally to any of the embodiments above, the filter system comprises a tangential flow filtration (TFF) filter.

Alternatively or additionally to any of the embodiments above, the filter system comprises filter capable of removing material having a mass in the range of 1 to 500 kDa.

Alternatively or additionally to any of the embodiments above, the filter system comprises filter having a pore size in the range of 0.1 to 5 microns.

Alternatively or additionally to any of the embodiments above, the one or more meningitis causing bacterial pathogen is a gram-negative bacterial pathogen.

Alternatively or additionally to any of the embodiments above, the one or more meningitis causing bacterial pathogen is Pseudomonas bacteria, Acinetobacter bacteria, Klebsiella bacteria, or combinations thereof.

Alternatively or additionally to any of the embodiments above, the one or more meningitis causing bacterial pathogen is multi-drug resistant.

Alternatively or additionally to any of the embodiments above, the one or more catheters comprises a single catheter comprising a first lumen with a first proximal port at the first location and a second lumen having a second distal port at the second location during at least a portion of a conditioning treatment.

Alternatively or additionally to any of the embodiments above, the one or more catheters comprises a first catheter inserted at the first location and a second catheter inserted at the second location during at least a portion of a conditioning treatment.

Alternatively or additionally to any of the embodiments above, the first catheter and the second catheter each comprise a single lumen.

Alternatively or additionally to any of the embodiments above, the flow rate is in a range from 0.04 ml/min to 30 ml/min.

Alternatively or additionally to any of the embodiments above, a volume of cerebrospinal fluid removed from the patient never exceeds 40 ml.

Alternatively or additionally to any of the embodiments above, wherein the second location is in the cervical cerebrospinal fluid space.

Alternatively or additionally to any of the embodiments above, the second location is in the ventricle of a brain.

Alternatively or additionally to any of the embodiments above, the second location is in a thoracic cerebrospinal fluid space.

Alternatively or additionally to any of the embodiments above, flow directions of removing and returning cerebrospinal fluid are periodically reversed so that CSF is returned to the first location and removed from the second location during a portion of the treatment.

Alternatively or additionally to any of the embodiments above, the flow reversal is a pulse to dislodge debris from removal or return ports.

Alternatively or additionally to any of the embodiments above, further comprising mixing the conditioned cerebrospinal fluid with endogenous cerebrospinal fluid as the conditioned cerebrospinal fluid is returned to the cerebrospinal fluid space.

Alternatively or additionally to any of the embodiments above, mixing comprises inducing a turbulent flow as the conditioned cerebrospinal fluid is returned.

Alternatively or additionally to any of the embodiments above, further comprising the step of isolating at least one of erythrocytes, hemoglobin, oxyhemoglobin, and endothelin.

Alternatively or additionally to any of the embodiments above, conditioning comprises one or more separation processes selected from the group consisting of biospecific affinity, immunoaffinity, cationic exchange, anionic exchange, hydrophobicity, and size exclusion.

Alternatively or additionally to any of the embodiments above, the conditioning step is performed externally to the patient's body.

A system for ameliorating a symptom of bacterial meningitis in a patient is disclosed. The system comprises: a catheter assembly having a first lumen with a distal port and a second lumen with a proximal port, said catheter being adapted to be introduced in a CSF space and said ports being spaced axially apart; a pump connectable between the first and second lumens to induce a flow of CSF therebetween; and a filter component connectable between the first and second lumens, the filter component configured to remove one or more meningitis causing bacterial pathogen from the CSF, to thereby condition the cerebrospinal fluid.

Alternatively or additionally to any of the embodiments above, the filter component is further configured to remove one or more endotoxins from the CSF.

Alternatively or additionally to any of the embodiments above, the filter component is further configured to remove one or more cytokines from the CSF.

Alternatively or additionally to any of the embodiments above, the filter component comprises a tangential flow filtration (TFF) filter.

Alternatively or additionally to any of the embodiments above, the filter component is capable of removing material having a mass in the range of 1 to 500 kDa.

Alternatively or additionally to any of the embodiments above, the filter component has a pore size in the range of 0.1 to 5 microns.

Alternatively or additionally to any of the embodiments above, the filter component is configured to remove one or more gram-negative bacterial pathogen.

Alternatively or additionally to any of the embodiments above, the filter component is configured to remove one or more of Pseudomonas bacteria, Acinetobacter bacteria, Klebsiella bacteria, or combinations thereof.

Alternatively or additionally to any of the embodiments above, the filter component is configured to remove one or more meningitis causing bacterial pathogen that is multi-drug resistant.

Alternatively or additionally to any of the embodiments above, the catheter assembly comprises a single catheter comprising a first lumen with a first proximal port and a second lumen having a second distal port.

Alternatively or additionally to any of the embodiments above, the catheter assembly comprises a first catheter and a second catheter.

Alternatively or additionally to any of the embodiments above, the first catheter and the second catheter each comprise a single lumen.

A method for treating meningitis is disclosed. The method comprises: removing cerebrospinal fluid from a patient with meningitis; filtering the cerebrospinal fluid with a filtration system, the filtration system including a 5kDa tangential flow filter, a 100 kDa tangential flow filter, or both; and returning the filtered cerebrospinal fluid to the patient.

Alternatively or additionally to any of the embodiments above, filtering the cerebrospinal fluid with a filtration system removes one or more meningitis causing bacterial pathogens from the cerebrospinal fluid.

Alternatively or additionally to any of the embodiments above, the one or more meningitis causing bacterial pathogens include gram-negative bacteria.

Alternatively or additionally to any of the embodiments above, the one or more meningitis causing bacterial pathogens include Pseudomonas bacteria, Acinetobacter bacteria, Klebsiella bacteria, or combinations thereof.

Alternatively or additionally to any of the embodiments above, filtering the cerebrospinal fluid with a filtration system removes one or more bacterial endotoxins from the cerebrospinal fluid.

Alternatively or additionally to any of the embodiments above, filtering the cerebrospinal fluid with a filtration system removes one or more neuroinflammatory agents from the cerebrospinal fluid.

Alternatively or additionally to any of the embodiments above, filtering the cerebrospinal fluid with a filtration system removes one or more meningitis causing fungal pathogens from the cerebrospinal fluid.

Alternatively or additionally to any of the embodiments above, the one or more meningitis causing fungal pathogens include Cryptococcus neoformans.

Alternatively or additionally to any of the embodiments above, filtering the cerebrospinal fluid with a filtration system removes one or more fungal antigens from the cerebrospinal fluid.

Alternatively or additionally to any of the embodiments above, filtering the cerebrospinal fluid with a filtration system removes one or more inflammatory agents from the cerebrospinal fluid.

Alternatively or additionally to any of the embodiments above, filtering the cerebrospinal fluid with a filtration system removes one or more cytokines from the cerebrospinal fluid.

Alternatively or additionally to any of the embodiments above, further comprising delivering a drug to the central nervous system.

Alternatively or additionally to any of the embodiments above, further comprising delivering a drug to the cerebrospinal fluid.

A system for treating fungal meningitis in a patient is disclosed. The system comprises: a catheter having a first lumen with a distal port and a second lumen with a proximal port, the catheter being designed to be introduced in a cerebrospinal fluid space and the ports being spaced axially apart; a pump connectable between the first and second lumens to induce a flow of cerebrospinal fluid therebetween; and a filter component connectable between the first and second lumens, the filter component configured to remove one or more meningitis causing fungal pathogens from the cerebrospinal fluid, to thereby condition the cerebrospinal fluid.

Alternatively or additionally to any of the embodiments above, the filter component is further configured to remove one or more fungal antigens from the cerebrospinal fluid.

Alternatively or additionally to any of the embodiments above, the filter component is further configured to remove one or more inflammatory agents from the cerebrospinal fluid.

Alternatively or additionally to any of the embodiments above, the filter component is further configured to remove one or more cytokines from the cerebrospinal fluid.

Alternatively or additionally to any of the embodiments above, the catheter is designed to aspirate cerebrospinal fluid from a patient at an aspiration rate, return conditioned cerebrospinal fluid to the patient at a return rate; wherein a waste rate is defined as the difference between the aspiration rate and the return rate; and wherein the waste rate is controlled to maintain physiologically appropriate cerebrospinal fluid volumes and intracranial pressure in the patient.

A system for treating fungal meningitis in a patient is disclosed. The system comprises: a catheter having a first lumen with a distal port and a second lumen with a proximal port, the catheter being designed to be introduced in a cerebrospinal fluid space and the ports being spaced axially apart; a pump connectable between the first and second lumens to induce a flow of cerebrospinal fluid therebetween; and a filter component connectable between the first and second lumens, the filter component configured to remove one or more meningitis causing bacterial pathogens from the cerebrospinal fluid, to thereby condition the cerebrospinal fluid.

Alternatively or additionally to any of the embodiments above, the filter component is further configured to remove one or more bacterial endotoxins from the cerebrospinal fluid.

Alternatively or additionally to any of the embodiments above, the filter component is further configured to remove one or more inflammatory agents from the cerebrospinal fluid.

Alternatively or additionally to any of the embodiments above, the filter component is further configured to remove one or more cytokines from the cerebrospinal fluid.

Alternatively or additionally to any of the embodiments above, the catheter is designed to aspirate cerebrospinal fluid from a patient at an aspiration rate, return conditioned cerebrospinal fluid to the patient at a return rate; wherein a waste rate is defined as the difference between the aspiration rate and the return rate; and wherein the waste rate is controlled to maintain physiologically appropriate cerebrospinal fluid volumes and intracranial pressure in the patient.

A system for treating meningitis is disclosed. The system comprises: a catheter having a first lumen with a distal port and a second lumen with a proximal port, the catheter being designed to be introduced in a cerebrospinal fluid space and the ports being spaced axially apart; a pump connectable between the first and second lumens to induce a flow of cerebrospinal fluid therebetween; and a filter component connectable between the first and second lumens, the filter component configured to remove one or more meningitis related pathogens from the cerebrospinal fluid, to thereby condition the cerebrospinal fluid.

A system for treating meningitis is disclosed. The system comprises: a catheter for removing one or more meningitis related pathogens, the catheter including a filtration system; and wherein the filtration system includes a 5kDa tangential flow filter, a 100 kDa tangential flow filter, or both.

The above summary of some embodiments is not intended to describe each disclosed embodiment or every implementation of the present disclosure. The Figures, and Detailed Description, which follow, more particularly exemplify these embodiments.

### Brief Description of the Drawings

The disclosure may be more completely understood in consideration of the following detailed description in connection with the accompanying drawings, in which:
FIG. 1 illustrates a Y-connector portion, a proximal subassembly, and a distal subassembly of a catheter according to certain implementations.
FIG. 2 illustrates a sectional view taken from the region of the catheter of FIG. 1 marked with cutting plane line A-A.
FIG. 3 illustrates a sectional view taken from the region of the catheter of FIG. 1 marked with cutting plane line B-B.
FIG. 4 illustrates an enlarged, detail view of a portion of the Y-connector of the catheter of FIG 1.
FIG. 5 illustrates the location of position markers on a catheter according to certain implementations.
FIG. 6 illustrates a sectional view taken from the region of the catheter of FIG. 5 marked with the cutting plane line J-J.
FIG. 7 illustrates a portion of a catheter near the joining of a proximal subassembly and a distal subassembly according to certain implementations.
FIG. 8 illustrates a portion of a proximal subassembly according to certain implementations.
FIG. 9 illustrates a detail view of the proximal subassembly of FIG. 8.
FIG. 10 illustrates a sectional view taken from the region of the proximal subassembly of FIG. 8 marked with the cutting plane line A-A.
FIG. 11 illustrates a detail view of a portion of the proximal subassembly of FIG. 9 taken from the view of line D-D.
FIG. 12 illustrates a sectional view taken from the region of the proximal subassembly of FIG. 8 marked with the cutting plane E-E.
FIG. 13 illustrates a portion of a distal subassembly according to certain implementations.
FIG. 14 illustrates a detailed portion of the distal subassembly of FIG. 13.
FIG. 15 illustrates a detailed portion of the distal subassembly of FIG. 13.
FIG. 16 illustrates a sectional view taken from the region of the distal subassembly of FIG. 13 marked with the cutting plane A-A.
FIG. 17 schematically illustrates an example pump system.

While the disclosure is amenable to various modifications and alternative forms, specifics thereof have been shown by way of example in the drawings and will be described in detail. It should be understood, however, that the intention is not to limit the disclosure to the particular embodiments described. On the contrary, the intention is to cover all modifications, equivalents, and alternatives falling within the scope of the disclosure.

### Detail Description

Cerebrospinal fluid (CSF) is a generally clear, colorless fluid that is produced in the ventricles, specifically the choroid plexuses, in the brain. The choroid plexus produces approximately 500 milliliters of CSF daily in order to accommodate flushing or recycling of CSF to remove toxins and metabolites, which happens several times per day. From the choroid plexus, CSF flows slowly through a channel (canal) into the space surrounding the brain and spinal column, and then into the body. CSF is found in the space between the pia mater and the arachnoid mater, known as the subarachnoid space. CSF is also found in and around the ventricular system in the brain, which is continuous with the central canal of the spinal cord. In the event of a stroke or other brain trauma, it can be desirable to remove the CSF from one location (e.g., the cervical region of the spine, or a brain ventricle), filter it, and return it to the CSF space at a second location (e.g., the lumbar region of the spine). However, accurate delivery of medical instruments to the CSF space can be challenging.

The present disclosure relates to removal, exchange and recirculation of cerebrospinal fluid (CSF). Devices, systems and methods disclosed herein are used to safely and efficiently navigate the space at and around the brain and spinal cord where the CSF flows through the body, also known as the CSF space. Specialized devices and systems are useful and sometimes necessary to navigate the CSF space due to the difficult points of entry and exit and the potentially life threatening consequences if a mistake is made. Increased safety and efficacy reduce time spent in the surgical suite and potential complications.

Neurapheresis may be understood to be the removal of materials (e.g., microrganisms, cells, viruses, foreign material, drugs, combinations thereof, and the like) from CSF. This and other therapeutic techniques can be used to treat a number of neurological diseases or conditions, such as Alzheimer's Disease, Parkinson's Disease, Huntington's Disease, Amyotrophic Lateral Sclerosis (ALS), Encephalitis from various causes, Meningitis from various causes, Guillain-Barré Syndrome (GBS), Multiple Sclerosis (MS), HIV-associated neurocognitive disorders, Spinal Cord Injury, Traumatic Brain Injury, cerebral vasospasm, stroke and other diseases or conditions.

The purification, conditioning, and/or compound removal schema can be tailored to a specific disease or group of diseases as suitable, including based on a number of features, such as size, affinity, biochemical properties, temperature, and other features. Purification schema may be based on diffusion, size-exclusion, ex-vivo immunotherapy using immobilized antibodies or antibody fragments, hydrophobic/hydrophilic, anionic/cationic, high/low binding affinity, chelators, antibacterial, anti-viral, anti-DNA/RNA/amino acid, enzymatic, and magnetic and/or nanoparticle-based systems. The system can be adjustable to a broad range of biologic parameters and flows.

With regard to a Neurapheresis system in particular, the disclosed system can be used to safely and quickly access the CSF space with minimal disturbance to the CSF flow. The systems and devices disclosed herein provide a safe and rapid flow circuit and provide filtration.

A Neurapheresis system should provide for the exchange, removal, and/or recirculation of CSF, safely and efficiently. The systems and devices disclosed herein may be used in a Neurapheresis system.

The systems and devices disclosed herein can be used to access the CSF space to remove the CSF from one location (e.g., the cervical region of the spine, or a brain ventricle), filter or otherwise treat it, and return it to the CSF space, including at a second location (e.g., the lumbar region of the spine), safely and efficiently. In various aspects, the systems and devices disclosed herein maintain the endogenous intracranial or intraspinal pressure within a physiological range, for example, from about 5 to about 20 mm Hg or from about 0 to about 10 mm Hg or from about -5 to about 10 mm Hg or from about -5 to about 25 mm Hg. The present system thus reduces spinal headache, for example, due to hydrocephalus (abnormal accumulation of CSF in the ventricles of the brain). The present system may also be used to reduce spinal headaches caused by low pressure (e.g., due to overdrainage, herniation, etc.). In some aspects, the system may include sensors within the catheter or within the flow circuit to detect clogs or blockages in the system, thereby providing closed loop pressure control. In various aspects, the systems and devices disclosed herein also help the system to perform efficiently by reducing or eliminating recirculating flow loops. The systems and devices maintain spacing between the inlet and outlet, for example, between about 10 cm to about 40 cm. In certain implementations, the spacing is between about 10 cm and about 30 cm. The inlets and outlets are located in places in the CSF space so that turning on the pump or otherwise creating positive or negative pressure in the system will not cause or encourage tissue being drawn into the catheter. In some aspects, the inlets and outlets are placed near the lumbar cervical cisterns to prevent tissue from being drawn into the catheter. In some aspects, there may also be multiple holes along the inlet and outlet for redundancy in case there is tissue blocking some number of holes. In certain implementations, a particular coil pitch of a coiled wire within the catheter may be selected in order to reduce kinking of the catheter. In certain aspects, the inlet-outlet spacing may be selected to be maximized while staying below the level of a cervical region of a patient. In certain aspects, the inlet-outlet spacing may be selected based on vertebral spacing. For example, the spacing may be selected so that the inlet-outlet spacing is between the lengths of approximately 5 vertebrae and approximately 12 vertebrae. In certain implementations, a spacing of approximately 10 vertebrae may be selected; however, other configurations (such as those described elsewhere in the specification) may be utilized. When designing such spacing, it may be assumed that a vertebra is approximately 2-3 cm in length, however, other measurements and designs may be used. In certain implementations, a particular size, shape, and/or other configuration of a lumen may be selected to facilitate catheter unblocking and/or the ability of the catheter to resist blockage. For example, a proximal outer diameter of a lumen of between approximately 0.060 inches and approximately 0.070 inches and a proximal inner diameter of between approximately 0.025 inches and 0.060 inches may be selected; however, other configurations (such as those described elsewhere in the specification) may be utilized.

The disclosed systems and devices are used to access the CSF space and may be used at any access point in the cervical (C1-C7), thoracic (T1-T12), or lumbar region (L1-L5) of the vertebral column. An access site in the cervical region may be used to access the ventricular system in the brain. In one embodiment, the system and device are used to access the lumbar region. In some embodiments, the inlets and outlets are located in places in the spine such that the drainage process will not cause tissue to be drawn into the catheter. For example, when a patient is lying on a table, entry may be made at a suitable angle, such as, for example, about 90 degrees, to access the spine. A traditional catheter must be pushed through a 90 degree bend at the L4-L6 region. The catheters and related delivery devices disclosed herein may be curved such that they can access and navigate this angled bend more easily and efficiently.

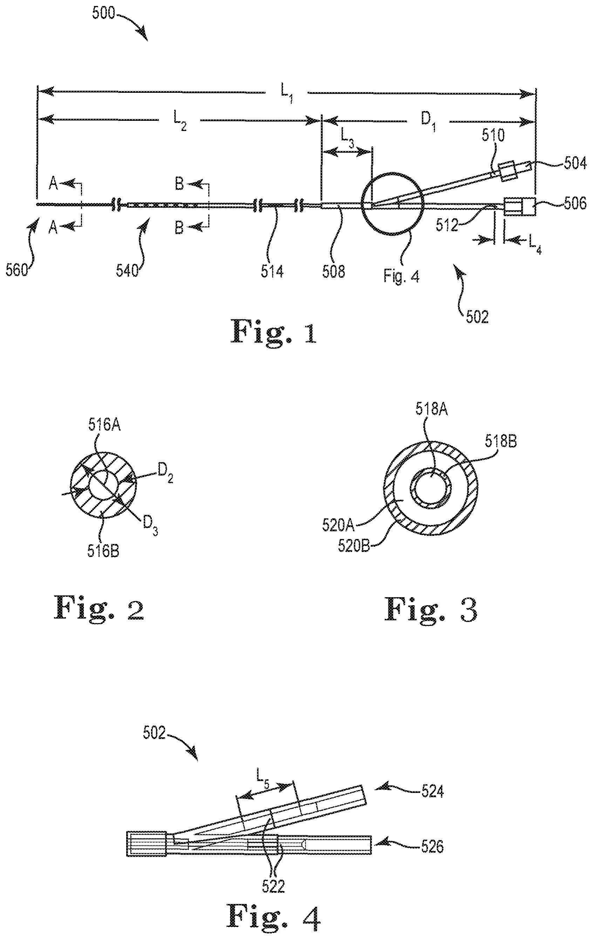

FIGS. 1-16 illustrate overall views, proximal subassembly views, and distal subassembly views of an embodiment of a catheter 500 according to certain implementations. FIG. 1 illustrates a Y-connector portion 502, a proximal subassembly 540, and a distal subassembly 560. The Y-connector portion 502 may include connectors 504, 506, features 508, 510, 512, position marker 514, and other components. The connectors 504, 506 may take various forms. For example, as illustrated, the connectors 504, 506 are female and male Luer-lock connectors, respectively. The features 508, 510, 512 may be strain relief and kink resistance features, for example, as described above with reference to strain relief and kink resistance feature 60. The feature 508 may be configured to allow flex or deformation of the catheter 500 at portions near a central meeting point of the Y-connector 502. The features 510, 512 may be configured to allow flex or deformation of the catheter 500 near the connectors 504, 506. In certain implementations, the features 510, 512 may be color coded to indicate to which lumen of a multi-lumen catheter, the connectors 504, 506, correspond. In certain embodiments, the features 508, 510, 512 may take the form of approximately 1/8" polyolefin heat shrink tubing. The position marker 514 may be a position marker as described above with reference to position marker 100.

The length L₁ of the catheter 500 may be approximately 1,300 mm with a working length L₂ of approximately 1,150 mm. The working length L₂ may be defined based on various use and design considerations. As illustrated, the working length L₂ is the distance from the distal end of the distal subassembly 560 to the distal end of the feature 508. The distance D₁ from the distal end of the feature 508 to the proximal end of the connector 506 may be approximately 150 mm. The feature 508 may have a length L₃ of approximately 35 mm and the features 510, 512 may have a length L₄ of approximately 7 mm. In certain implementations, the catheter 500 may have a length L₁ of between approximately 400 mm and approximately 1200 mm, with the working length L₂ and other measurements changed accordingly at varying scales.

FIG. 2 illustrates a sectional view taken from the region of the catheter 500 marked with cutting plane line A-A. This view illustrates a lumen 516A defined by a wall 516B. The characteristics and properties of the lumen 516A and wall 516B may be similar to the other walls and lumens described herein. As illustrated, the wall 516B has an inner diameter D₂ of approximately 0.54 mm and an outer diameter D₃ of approximately 1.14 mm.

FIG. 3 illustrates a sectional view taken from the region of the catheter 500 marked with cutting plane line B-B. This view illustrates a lumen 518A defined by an inner wall 518B and a lumen 520A defined by the space between the inner wall 518B and an outer wall 520B. The characteristics and properties of the lumens 518A, 520A and the walls 518B, 520B may be similar to the other walls and lumens described herein. The inner wall 518B may have an inner diameter D₄ of approximately 0.56 mm and an outer diameter D₅ of approximately 0.71 mm. The outer wall 520B may have an inner diameter of approximately 1.32 mm and an outer diameter of approximately 1.689 mm.

FIG. 4 illustrates an enlarged, detail view of a portion of the Y-connector 502 according to certain implementations, including tubes 522, first branch 524, and second branch 526. The tubes 522 may be hypotubes or other lengths of tubing. The tubes 522 may have a length L₅ of approximately 10 mm. In certain implementations, the first branch 524 may place the connector 504 in fluid connection with the lumen 520A and the second branch 526 may place the connector 506 in fluid connection with the lumen 518A.

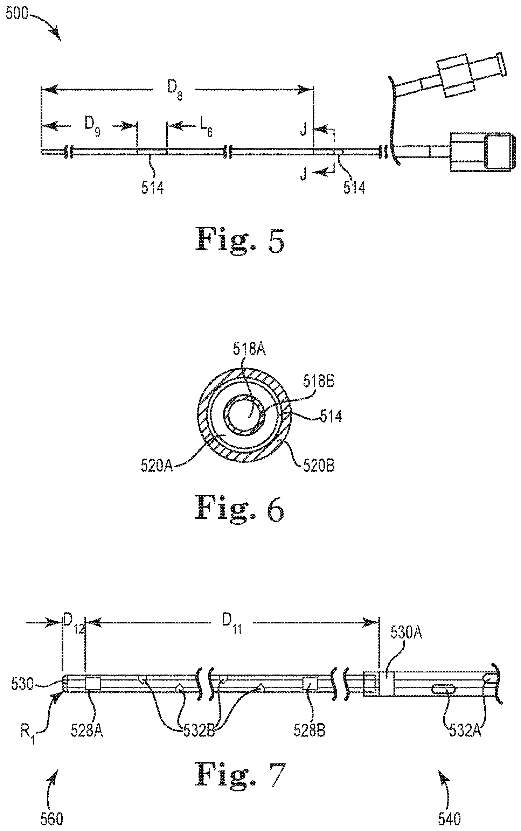

FIG. 5 illustrates the location of two position markers 514 on the catheter 500. The distal end of the first position marker 514 is located a distance D₉ of approximately 450 mm away from the distal end of the catheter 500. The distal end of the second position marker 514 is located a distance D₈ of approximately 550 mm away from the distal end of the catheter 500. The length L₄ of the position markers 514 is approximately 10 mm. In certain implementations, the bands and/or position markers (such as position markers 514) may comprise PET heat shrink tubing.

FIG. 6 illustrates a sectional view taken from the region of the catheter 500 marked with the cutting plane line J-J. This view illustrates an embodiment wherein an outer portion of the position marker 514 is substantially adjacent to an inner portion of the wall 520B. Accordingly, in this portion of this embodiment, the lumen 520A is defined by the outer portion of the wall 518B and the inner portion of the position marker 514. As illustrated, the outer wall 520B has an outer diameter D₁₀ of approximately 1.75 mm. In other instances, the position marker 514 may be disposed along the outer portion of the wall 520B, along the outer portion of the wall 518B, or along another region of the catheter 500.

FIG. 7 illustrates a portion of the catheter 500, including bands 528A, 528B, and 530A, openings 532A and 532B, and a radiused tip 530. The distal portion of the band 530A may be located a distance D₁₁ of approximately 300 mm away (e.g., or more or less, depending on the size/height of the patient) from the distal portion of the band 528A. This spacing may help to reduce local recirculation and/or help avoid sensitive nerve structures in the cervical spine. The distal end of the band 528A may be located a distance D₁₂ of approximately 2 mm away from the distal end of the radiused tip 530. The radiused tip may have a radius R₁ of approximately 0.28 mm.

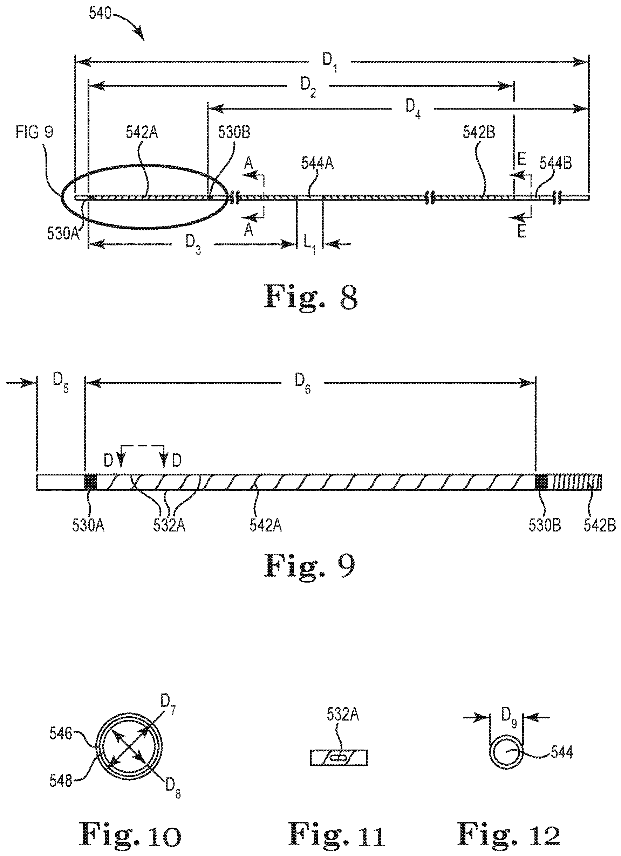

FIG. 8 illustrates a portion of the proximal subassembly 540. As illustrated, the distance D₁ from the distal end of the proximal subassembly 540 to the proximal end of the proximal subassembly 540 is approximately 893 mm. The distance D₂ from the distal end of a marker band 544B to a distal end of a band 530A is approximately 248 mm. A distance D₄ from a proximal end of the proximal subassembly 540 to the distal end of a band 530B is approximately 845 mm. A distance D₃ from a distal end of the marker band 544A to a distal end of the band 530A is approximately 148 mm. The marker bands 544A, 544B may have a length L₁ of approximately 10 mm. A portion of the proximal subassembly 540 may comprise coiled wire 542B having a coil pitch of approximately 0.018". A portion of the proximal subassembly 540 may comprise coiled wire 542A having a coil pitch of approximately 0.095". In certain implementations, the wires 542A, 542B may comprise approximately 0.003" round wire spool of 304V spring temper material.

In certain implementations, the proximal subassembly 540 of the catheter 500 may have an outer diameter of between approximately 0.06" and approximately 0.07". This configuration may maximize the size of the catheter between layers of tissue to enable a desired level of drainage and/or suction without collapse. The thickness of the proximal subassembly 540 and other sections of the catheter 500 may be a function of a design of one or more layers of coil and sheath. The thickness may affect the stiffness and pushability of the catheter 500 and kink-resistance. In certain implementations, the diameter of an inner lumen of the catheter 500 (such as the diameter of a lumen of the proximal subassembly 540) may be chosen to provide optimum drainage and/or suction given the constraints of particular anatomy or procedures. For example, the minimum diameter of a proximal inner lumen may be chosen to be between approximately 0.025" and approximately 0.060".

FIG. 9 illustrates a detail view of the proximal subassembly 540 of FIG. 8. As illustrated, a portion of the proximal subassembly 540 defines a plurality of openings 532A (e.g. 2, 3, 4, 5, 6, 7, 8, 9, 10 11, 12. 13 14. 15, 16, or more openings 532A). The openings 532A may be in fluid connection with a lumen 520A of the catheter 500 and, in at least some instances, may be arranged on opposing "top" and "bottom" sides of the catheter 500. The openings 532A may be spaced with 2 coil pitch spacing of the wire 542A. The distance D₆ between the distal end of the band 530B and the distal end of the band 530A is approximately 45 mm. A distance D₅ from the distal end of the band 530A to the distal end of the proximal subassembly 540 may be approximately 3 mm. In certain implementations, the bands 530A, 530B may comprise a radiopaque band having an inner diameter of approximately 0.061" and an outer diameter of approximately 0.064".

FIG. 10 illustrates a sectional view taken from the region of the proximal subassembly 540 marked with the cutting plane line A-A, including a liner 546 and tubing 548. The liner 546 and the tubing 548 may be arranged such that the tubing 548 is within the liner 546. The coil 542A may be disposed between the liner 546 and the tubing 548. In certain implementations, the liner 546 may comprise approximately 0.001" WT PTFE liner. The tubing 548 may comprise approximately 0.004" WT polyether block amide tubing. The outer diameter D₇ of the combination tubing 548 and liner 546 may be approximately 1.69 mm. The inner diameter D₈ of the same may be approximately 1.32 mm.

FIG. 11 illustrates a detail view of a portion of the proximal subassembly 540 taken from the view of line D-D and illustrating one of the openings 532A. The illustrated opening 532A has dimensions of approximately 1.57 mm by approximately 0.56 mm. In at least some instances, the openings 532A may be oval in shape. Other shapes are contemplated. The shape of the openings 532A may be the same along the length of the proximal subassembly 540 or the shape of the openings 532A may differ along the length of the proximal subassembly 540. In at least some instances, the openings 532A may be larger than the openings 532B.

FIG. 12 illustrates a sectional view taken from the region of the proximal subassembly 540 marked with the cutting plane E-E. As illustrated the outer diameter D₉ this portion, inclusive of marker band 544 is approximately 1.75 mm.

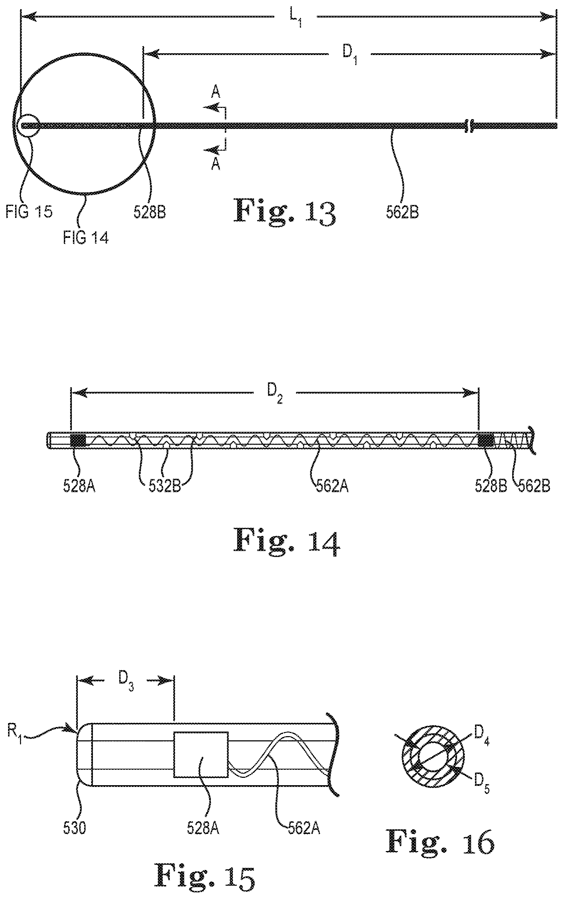

FIG. 13 illustrates a portion of the distal subassembly 560. The length L₁ of the distal subassembly 560 may be approximately 302 mm. The distance D₁ from the proximal end of the distal subassembly 560 to the distal end of a band 528B is approximately 270 mm. A portion of the distal subassembly 560 may comprise a coiled wire 562B may have a coil pitch of approximately 0.032". This and other portions of the catheter 500 may comprise approximately 0.003" WT nylon 12 tubing having an inner diameter of approximately 0.022" and approximately 0.007" WT PEBAX tubing having an inner diameter of approximately 0.04".

FIG. 14 illustrates a detailed portion of the distal subassembly 560, including the band 528A, a plurality of openings 532B, the band 528B, a wire 562A, and the wire 562B. In certain implementations, the wires 562A, 562B may be different portions of the same wire or may be separate sections of wire. As illustrated, the wire 562A and 562B may be separated by band 528B. The wire 562A may have a coil pitch of approximately 0.065". The wires 562A, 562B may be disposed between layers of the distal subassembly 560 and may comprise approximately 0.003" round wire spool 304V spring temper material. The openings 532B may be spaced with 2 coil pitch spacing and arranged on a top and a bottom portion of the catheter 500 and made a fluid connection with an inner lumen 516A of the catheter 500. In at least some instances, the openings 532B may be round or substantially round. Other shapes are contemplated. The shape of the openings 532B may be the same along the length of the distal subassembly 560 or the shape of the openings 532B may differ along the length of the distal subassembly 560. A distance D₂ between the distal end of the band 528B and the distal end of the band 528A may be approximately 30 mm. The wire 562A may be disposed within this region. The bands 528A, 528B may have an inner diameter of approximately 0.032" and an outer diameter of approximately 0.034". The bands 528A, 528B may include a radiopaque material (e.g., the bands 528A, 528B may comprise a material such as PT/10%IR). The bands 528A, 528B may be disposed between the layers of the distal subassembly 560.

FIG. 15 illustrates a detailed portion of the distal subassembly 560, including the radiused tip 530, the band 528A, and the wire 562A. The distance from the distal end of the band 528A and the distal end of the radiused tip 530 is approximately 2 mm. The radiused tip may have a radius R₁ of approximately 0.28 mm.

FIG. 16 illustrates a sectional view taken from the region of the distal subassembly 560 marked with the cutting plane A-A. As illustrated, this section of the distal subassembly 560 has an outer diameter of approximately 1.14 mm and an inner diameter of approximately 0.53 mm.

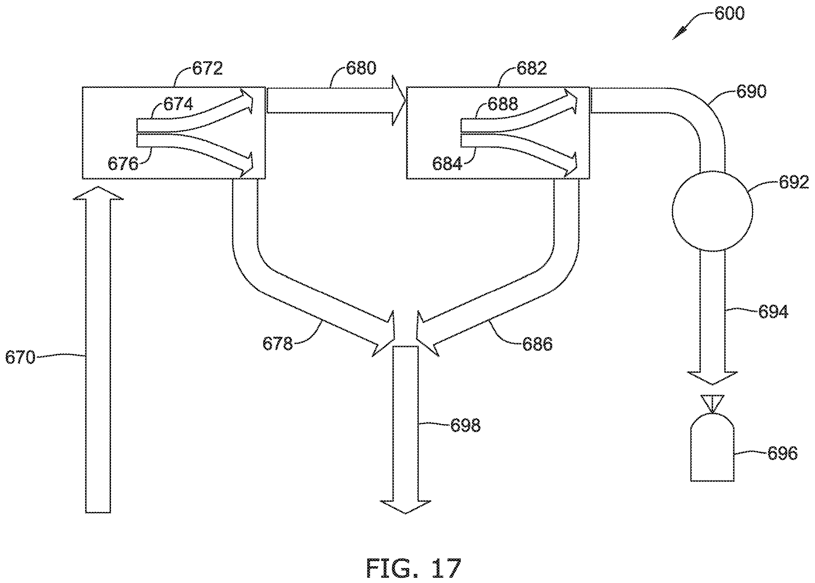

FIG. 17 schematically depicts a pump/filtration system 600 that may be utilized with the catheter 500. The catheter 500 may connect to an inlet 670 of the pump/filtration system 600. For example, the connector 504 may connect to the inlet 670 either directly or through an intermediate tube or mechanism. The inlet 670 may lead to a first filter 672. In some instances, the first filter 672 is a tangential flow filter. For example, the first filter 672 may include a 5kDa tangential flow filter (TFF), a 100kDa TFF, a 0.2 µm TFF, a 0.45 µm TFF, or the like. In some instances, the first filter 672 may include a dead-end filter (e.g., 5kDa dead-end filter). In some instances the first filter 672 may include an electro-filter (e.g., a filter that excludes materials based on charge). In some instances, only one filter (e.g., the first filter 672) may be utilized. For example, the first filter 672 may be a 5kDa filter and the first filter 672 may be the only filter. Clean CSF 676 may follow pathway 678. CSF waste 674 may follow pathway 680. The waste pathway 680 may lead to a second filter 682. In some instances, the second filter 682 is a tangential flow filter. For example, the second filter 682 may include a 5kDa TFF, a 100kDa TFF, a 0.2 µm TFF, a 0.45 µm TFF, or the like. In some instances, the second filter 682 may include a dead-end filter (e.g., 5kDa dead-end filter). In some instances the second filter 682 may include an electro-filter. In at least some instances, the first filter 672 and the second filter 682 are the same size and/or type (e.g., both the first filter 672 and the second filter 682 are 100kDa TFF). In other instances, the first filter 672 and the second filter 682 differ (e.g., the first filter 672 is a 5kDa filter and the second filter 682 is a 100kDa TFF filter). Clean CSF 684 may follow pathway 686. CSF waste 688 may follow pathway 690. A valve or flow metering mechanism 692 may be disposed along the waste pathway 690, before terminating at pathway 694 and a collection apparatus 696. Pathways 678 and 686 may merge into a return outlet 698, which may connect to the connector 506 of the catheter 500 (e.g., either directly or through an intermediate tube).

In use, the catheter 500 may be disposed within the cerebrospinal space (e.g., such as along lumbar cerebrospinal space). CSF may be removed/aspirated using the catheter 500 (e.g., via the lumen 520A) and the pump/filtration system 600. The aspirated fluid may be filtered using the pump/filtration system 600 and the filtered/conditioned CSF may be returned to the patient using the catheter 500 (e.g., via the lumen 518A) and the pump/filtration system 600. In some instances, a second catheter 500 (that may be similar in form and function to the catheter 500) may be disposed in a portion of the cranial CNS such as within a ventricle. The second catheter 500 may be used to remove/aspirate cerebrospinal fluid from a cranial region (e.g., a ventricle), condition/filter the cerebrospinal fluid using the pump/filtration system 600, and return the conditioned/filtered cerebrospinal fluid to a region at or adjacent to the cranial region. In some of these and in other instance, the second catheter 500 may be used to infuse a drug (e.g., a chemotherapy drug such as methotrexate) into the cranial region. The catheter 500 (e.g., in the cerebrospinal space) and the second catheter 500 (in the ventricle) may be used together or they may be used alternately. Using a catheter 500 in both the cerebrospinal space and in the ventricle, both for aspiration and infusion, may form a cranial-lumbar loop that may improve circulation of cerebrospinal fluid throughout the CNS.

The catheter 500 (along with the pump/filtration system 600) may be used to treat a number of conditions. Some of the contemplated conditions include cancer. For example, Leptomeningeal Metastases (LM) is a condition in which cells from a primary solid or hematological tumor metastasize, invade the subarachnoid space (SAS), and spread throughout the cerebrospinal fluid (CSF), resulting in seeding of the leptomeninges along the surface of the central nervous system (CNS). LM represents a late event of cancer progression and the most frequent symptoms include multiple cranial nerve deficits, motor deficits, altered mental status, headache, and radicular pain. The incidence of LM is estimated at 3-5% of cancer patients and has been increasing, due to longer overall survival in cancer patients. LM presents a difficult challenge in metastatic cancer treatment plans, resulting in a devastating prognosis and median survival of 4 months because of lack of effective access and therapies. Systemic therapy with anti-cancer drugs including methotrexate (MTX), cytarabine and thiotepa are not as effective due to poor penetration of the blood-brain barrier (BBB). Intrathecal (IT) drug delivery systems, including Ommaya reservoirs, have been associated with longer overall survival; however, they require repeated injections and rely on passive diffusion. Future therapies that target the entire CNS and enhance the distribution of IT drugs could further improve survival. CSF is produced at approximately 20 ml/hr, with a total volume of ~150 ml, resulting in a turnover, on average, of three times per day. The production rate of CSF is independent of intracranial pressure (ICP). As LM can block the outflow paths of CSF, patients are at serious risk of hydrocephalus and elevated ICP. Additionally, the relative isolation of the CSF by the BBB and blood-CSF barriers, presents a unique environment for tumor survival.

The catheter 500 may have the ability to rapidly clear a number of CSF pathogens and cells, as well as to enhance drug delivery in the CSF. For example, the catheter 500 may be used to improve the LM outcome by 1) enhanced exposure and circulation of specific anticancer agents (MTX delivered through an Ommaya reservoir, through the catheter 500, or both) throughout the SAS, (2) local filtering of CSF to remove cancer-spreading circulating tumor cells (CTCs), (3) control of ICP via CSF drainage, (4) filtration of tumor cells (e.g., living and/or dead tumor cells that may clog the natural reabsorption of the CSF via the arachnoid granulations and lymphatic system). The catheter 500 may also be used to reduce the concentration of a drug (e.g., a chemotherapy agent such as methotrexate) in the CSF (e.g., in order to remove excess drug, reduce toxicity, etc.).

As alluded to herein, treatment methods are contemplated that include infusing a chemotherapy agent into the patient. In some instances, the chemotherapy agent is methotrexate. Other chemotherapy agents are contemplated. The chemotherapy agent may be infused into the CNS via an Ommaya reservoir (and/or or a similar device including, for example, a Rickham device) implanted in the ventricles of the patient, as is standard of care in these patients. In addition or in the alternative, the chemotherapy agent may be infused into the patient using the catheter 500. For example, the chemotherapy agent may be added to the return outlet 698, to one of the ports of the catheter 500, via a separate device disposed agent to the catheter 500, or in another suitable manner. The circulation of CSF by the catheter 500 and pump/filtration system 600 may help to circulate the chemotherapy agent throughout the cerebrospinal space and/or the CNS.

Another contemplated condition that the catheter 500 may be used to treat is Amyotrophic Lateral Sclerosis (ALS). For example, the pathology of ALS may be correlated with overstimulation of glutamatergic functions/pathways with a corresponding excitotoxicity, increased calcium levels, and/or the generation of reactive oxygen species. Oxidative stress may be involved in pathological mechanisms of ALS via cell death-related release of pro-oxidative compounds and redox-active iron, mitochondrial dysfunction, inflammation, and excitotoxicity. The catheter 500 and pump/filtration system 600 may be used to help reduce/clear the CSF of oxidative and/or inflammatory agents (e.g., including free radicals, cytokines, chemokines, white blood cells) such as those correlated with the pathology of ALS. Some examples of materials that may be reduced/removed as part of treating ALS may include one or more of insoluble superoxide dismutase-1 (SOD1), glutamate, neurofilament protein, and anti-GM1 ganglioside antibodies.

In some instances, the oxidative and/or inflammatory agents may carry an electrical charge. Removal of such materials may be enhanced utilizing electrofiltration (e.g., a filter having an electrical charge). Accordingly, in at least some instances, the first filter 672, the second filter 682, or both may include an electrically charged filter (electrofilter). In some of these and in other instances, the first filter 672, the second filter 682, or both may include an immunoaffinity column, a size exclusion column, an anionic exchange column, a cationic exchange column, and a Protein A or Protein G column.

In addition to removing CSF-borne pathological mediators correlated with ALS, the catheter 500 and pump/filtration system 600 may also be used to deliver one or more drugs to the CSF. Such treatments may help further reduce oxidative and/or inflammatory agents. In some instances, the drug may be added to the return outlet 698, to one of the ports of the catheter 500, via a separate device disposed agent to the catheter 500, or in another suitable manner. The circulation of CSF by the catheter 500 and pump/filtration system 600 may help to circulate the drug throughout the cerebrospinal space and/or the CNS. Some example drugs that may be utilized may include riluzole, edaravone, or the like.

Another contemplated condition that the catheter 500 may be used to treat is herpes simplex encephalitis (HSE). HSE is known to cause severe neuroinflammation, cerebral edema and hemorrhagic necrosis with resultant increases in intracranial pressure (ICP). While medical management has been standardized, aggressive combined medical and surgical management including decompressive craniectomy and/or temporal lobectomy is often performed due to uncontrolled ICP, neuroinflammation and cerebral edema. The production of reactive oxygen species (ROS) are also believed to be a component of natural defenses to viral infection. However, the lipid-rich environment of the CNS may be susceptible to oxidative damage. Thus, oxidative damage can be correlated with HSE infection.

The catheter 500 and pump/filtration system 600 may be used to remove oxidative and/or inflammatory agents (e.g., including free radicals, cytokines, chemokines, white blood cells) such as those correlated with the pathology of HSE. In some instances, the oxidative and/or inflammatory agents may carry an electrical charge. Removal of such materials may be enhanced utilizing electrofiltration (e.g., a filter having an electrical charge). Accordingly, in at least some instances, the first filter 672, the second filter 682, or both may include an electrically charged filter (electrofilter).

Another contemplated condition that the catheter 500 may be used to treat is human immunodeficiency virus (HIV) and/or acquired immune deficiency system (AIDS). HIV infection of the CNS can lead to a number of complications including meningitis, acute inflammatory polyneuropathy (AIDP), immune reconstitution inflammatory syndrome (IRIS) - initiated by introduction of antiretroviral therapy, chronic inflammatory polyneuropathy (CIDP), distal symmetric polyneuropathy (DSP), progressive multifocal leuko-encephalopathy (PML), and HIV-associated neurocognitive disorders (HAND). The catheter 500 and pump/filtration system 600 may be designed to filter/reduce/remove a number of different strains of HIV from the CNS. This can reduce viral load in the CSF and/or reduce complications associated with HIV infection in the CNS. In addition, the catheter 500 and pump/filtration system 600 may be designed to filter/reduce/remove a number of different inflammatory agents associated with HIV from the CNS.

Another contemplated condition that the catheter 500 may be used to treat is multiple sclerosis (MS). Two subtypes, Clinically Isolated Syndrome (CIS) and Relapsing-Remitting Multiple Sclerosis (RRMS), represent the disease absent progression, while Primary Progressive (PPMS) and Secondary Progressive (SPMS) represent patients with progressive disease from the start or after RRMS, respectively. Neuroinflamation leading to multifocal lesion formation, demyelination, axonal damage and consequent neurodegeneration are hallmarks of the disease. Current treatments may be classified as including (1) anti-inflammatory naturally-occurring molecules (IFN-beta), (2) molecules that stimulate anti-inflammatory (glatiramer acetate) or inhibit autoreactive (teriflunomide) cell proliferation, (3) immunosuppressive monoclonal antibodies (natalizumab), (4) molecules that bind transcription factors to enhance anti-inflammatory mechanisms or suppress pro-inflammatory ones (dimethyl fumarate), and (5) agents that inhibit egress of lymphocytes from lymphoid tissue to the CNS (fingolomod). In some instances, the catheter 500 and pump/filtration system 600 may be designed to filter/reduce/remove a number of different inflammatory agents associated with MS including immune cells (immunoglobins, neutrophils, lymphocytes, monocytes, and the like), oxidative and/or inflammatory agents (e.g., including free radicals, cytokines, chemokines, white blood cells) such as those correlated with the pathology of MS, and the like. This can help treat MS and/or improve the symptoms thereof.

Another contemplated condition that the catheter 500 may be used to treat is Guillain-Barré syndrome (GBS). GBS is the most common cause of acute paralytic neuropathy worldwide. Acute motor axonal neuropathy (AMAN) and acute inflammatory demyelinating polyneuropathy (AIDP) are the main phenotypes. GBS may arise in individuals through a combination of host genetic and environmental factors, and preceding infection by pathogens including *Campylobacter jejuni* and Zika virus. Prevailing mechanisms of action implicate molecular mimicry of foreign antigen and gangliosidic residues resulting in the development of autoantibodies which recognize myelin or axonal components and initiate an inflammatory immune response including macrophage and/or lymphocytic infiltration, complement deposition, and cytokine production. CSF analysis shows elevated protein (>400 mg/L) and the absence of pleocytosis in 90% of patients. Elevated levels of neuroinflammatory cytokines and other proteins involved in the pathology have been noted, though specific immunological protein profiles of GBS CSF are heterogenous. In some of these and in other instances, a second catheter (e.g., which may be similar in form and function to the catheter 500, an Ommaya reservoir, or the like) may be used to infuse a drug into the cranial region.

Current treatments for GBS may include plasma exchange (PE) or intravenous immunoglobulins (IVIg) with supportive care. Based on protein abnormalities of the CSF in GBS patients, including elevated levels of inflammatory cytokines TNF-α and IL-6⁷, anti-ganglioside antibodies, and activated complement components, filtration of CSF to reduce/remove inflammatory may help to reduce GBS systems and/or treat GBS. In some instances, the catheter 500 and pump/filtration system 600 may be designed to filter/reduce/remove a number of different inflammatory agents associated with GBS including immune cells (immunoglobins, neutrophils, lymphocytes, monocytes, and the like), oxidative and/or inflammatory agents (e.g., including free radicals, cytokines, chemokines, white blood cells) such as those correlated with the pathology of GBS, and the like. This can help treat GBS and/or improve the symptoms thereof. In some instances, the catheter 500 and pump/filtration system 600 may include a 5 kDa filter when used for treating GBS. Other filter sizes are contemplated including those disclosed herein. For example, the catheter 500 and pump/filtration system 600 may include a 5kDa tangential flow filter, a 100 kDa tangential flow filter, an electrofilter, or a combination thereof.

Another contemplated condition that the catheter 500 may be used to treat is meningitis. Bacterial meningitis occurs when pathogenic bacteria enter the subarachnoid space and cause a pyogenic inflammatory response. Gram-negative bacterial meningitis (GBM) is a devastating condition that occurs when gram-negative bacteria invade the central nervous system (CNS). There are 30,000 US cases and over 1 million cases of GBM worldwide annually. When bacterial infections are manifested as GBM, it creates an extreme burden of mortality, often exceeding 30%, and morbidity to the patient and is very difficult for clinicians to treat, even when caused by bacteria susceptible to standard antibiotics. It is seen most commonly in children or immunocompromised patients, such as those with HIV, post organ-transplant or post-neurosurgical procedures. Current treatment guidelines include intravenous cephalosporins or carbapenems or polymycin for at least 10 days to 2 weeks. In the presence of gram-negative enteric bacterial meningitis, classically occurring around trauma and neurosurgical procedures, highly resistant bacteria can cause disease. Antibiotics like aminoglycosides and polymycins are considered for treatment but the therapeutic-toxic ratio is poor for these agents with systemic use in CNS disease and there may be no optimal treatments.

Three key gram-negative pathogens that have been deemed critical priority include *Pseudomonas, Acinetobacter* and *Klebsiella (PAK).* These gram-negative bacteria can cause severe and often deadly infections such as pneumonia, bloodstream infections and, specifically, nosocomial meningitis. These bacteria have become resistant to a large number of antibiotics, including carbapenems and third generation cephalosporins - the best available antibiotics for treating multidrug-resistant bacterial meningitis. The world health organization acknowledges that multi-modal approaches are needed and that waiting any longer will cause further public health problems and dramatically impact patient care and survival. This raises the very real possibility of GBM infections that are untreatable by presently available antibiotics. This return to the pre-antibiotic era has unfortunately become a reality in many parts of the world.

Reduction in CSF organism burden is the single most important factor impacting survival and is linked to a better overall clinical outcome. The rapid reduction in CSF organism burden is important, with sterilization of the CSF in the first 24 hours. Optimization of the antibiotic effect depends directly on the organism load that is present and on the direct activity of antibiotic therapy being started early in infection. Determining which antibiotic agent will be most effective is becoming increasingly more difficult in the face of drug-resistant bacteria such as *PAK.* Clinical data for new antibiotics for bacterial meningitis simply have not kept pace with the rise of resistance, and the development of new therapeutic approaches is urgently needed. Additionally, experimental animal models have shown that outcome from bacterial meningitis are related to the severity of inflammation in the subarachnoid space (SAS) and could potentially be improved by modulation of the inflammatory response.

The catheter 500 and pump/filtration system 600 may provide an innovative new treatment option that provides direct access to the CSF and creates active circulation combined with targeted pathogen removal. This may provide a novel therapeutic approach that rapidly reduces CFUs and CSF bacterial burden and translates to reduced morbidity and mortality from bacterial meningitis.

Accordingly, the present methods provide for ameliorating or reducing the symptoms of bacterial meningitis by reducing or eliminating the presence of one or more of bacterial pathogens and/or their associated endotoxins and/or cytokines in the CSF using the catheter 500 and pump/filtration system 600. The methods comprise removing CSF from a patient, removing at least one of the bacterial pathogens, and/or endotoxins associated with the bacterial pathogens, and/or cytokines from the CSF, and returning the endogenous CSF to the patient, wherein the removing and returning steps are performed concurrently during at least a portion of the treatment. In some embodiments, the cytokines are selected from the group consisting of IL-1ra, IL-6, TNF, CRP, and CXCL10, or combinations thereof.

In some of these and in other instances, the methods provide for ameliorating or reducing the symptoms of bacterial meningitis by introducing the catheter 500 through a spinal access site into a spinal CSF space of a patient, advancing the catheter 500 through the spinal CSF space toward the brain so that the openings 532A and 532B are disposed within the CSF space and spaced-apart by a preselected distance or adjusted to an appropriate distance, withdrawing CSF through at least some of the openings in the catheter 500 (e.g., the openings 532A), removing at least one of bacterial pathogens and/or their associated endotoxins and/or cytokines from the withdrawn CSF with the pump and filtrations system 600 (thereby conditioning the CSF), and returning the conditioned CSF through the other of the openings in the catheter 500 (e.g., the openings 532B).

Fungal meningitis (FM) is an infection of the meninges of the central nervous system that manifests from the dissemination of any major fungal pathogen into the subarachnoid space (SAS) via the cerebrospinal fluid (CSF). Cryptococcal Meningitis (CM) is caused by Cryptococcus neoformans and is the most common cause of fungal meningitis in adults. Other agents causative of fungal meningitis include: C. Gattii, Blastomyces, Histoplasma, Coccidioides. Treatment for CM is based on an induction, consolidation, and maintenance approach with antifungals and is well defined elsewhere, but is associated with continued high morbidity and mortality. Drug discovery programs are limited by poor penetration of the Blood Brain Barrier (BBB). Because of this, we developed an alternative catheter-based extracorporeal filtration system (Neurapheresis Therapy) for the filtration of infected CSF. Here we describe the in vitro characterization of Neurapheresis Therapy as an alternative mechanical intervention for filtration of C. neoformans cells, polysaccharide antigen, and inflammatory mediators from infected CSF.

The catheter 500 and pump/filtration system 600 may provide an innovative new treatment option that provides direct access to the CSF and creates active circulation combined with targeted pathogen removal. This may provide a novel therapeutic approach that rapidly reduces CFUs and CSF fungal burden and translates to reduced morbidity and mortality from fungal meningitis. In at least some instances, the catheter 500 and pump/filtration system 600 may include one or more filters (e.g., the filters 672/682) designed to exclude the passage of fungi therethrough such as C. neoformans. In some of these and in other instances, the catheter 500 and pump/filtration system 600 may include one or more filters (e.g., the filters 672/682) designed to exclude fungi (e.g., C. neoformans), associated antigens, and/or inflammatory agents. In at least some instances, a single pass of CSF through a 5kDa TFF and/or a 100kDa TFF may be sufficient to exclude C. neoformans or other reduce the CFUs of C. neoformans in the CSF. In addition, a 5kDa TFF and/or a 100kDa TFF may be sufficient to exclude or otherwise reduce C. neoformans antigen from the CSF. Furthermore, a 5kDa and/or 100kDa TFF may also exclude a number of neuroinflammatory agents such as IL-1ra, IL-6, TNF, CRP, and/or CXCL 10/IP-10 from the CSF.

Accordingly, the present methods provide for ameliorating or reducing the symptoms of fungal meningitis by reducing or eliminating the presence of one or more of fungal pathogens and/or their associated antigens (e.g., Cryptococcal antigen) and/or cytokines in the CSF using the catheter 500 and pump/filtration system 600. The methods comprise removing CSF from a patient, as described herein; removing at least one of the fungal pathogens, and/or antigens associated with the fungal pathogens, and/or cytokines from the CSF, and returning the endogenous CSF to the patient, wherein the removing and returning steps are performed concurrently during at least a portion of the treatment. In some embodiments, the cytokines are selected from the group consisting of IL-1ra, IL-6, TNF, CRP, and CXCL10, or combinations thereof. The fungus/fungi and/or antigens and/or cytokines can be removed from the CSF using one or more filtration system. A 5kDa and/or 100kDa TFF may also exclude a number of neuroinflammatory agents such as IL-1ra, IL-6, TNF, CRP, and/or CXCL 10/IP-10.

In some of these and in other instances, the methods provide for ameliorating or reducing the symptoms of fungal meningitis by introducing the catheter 500 through a spinal access site into a spinal CSF space of a patient, advancing the catheter 500 through the spinal CSF space toward the brain so that the openings 532A and 532B are disposed within the CSF space and spaced-apart by a preselected distance or adjusted to an appropriate distance, withdrawing CSF through at least some of the openings in the catheter 500 (e.g., the openings 532A), removing at least one of fungal pathogens and/or their associated antigens and/or cytokines from the withdrawn CSF with the pump and filtrations system 600 (thereby conditioning the CSF), and returning the conditioned CSF through the other of the openings in the catheter 500 (e.g., the openings 532B).

In at least some instances, the catheter 500 and pump/filtration system 600 may be used to deliver drugs to portions of the CNS. For example, some treatments for CM may include the administration of intravenous and oral antifungals such as amphotericin B (AmB) and flucytosine. Generally, intrathecal (IT) AmB boluses may be associated with neurotoxic drug concentrations near the injection site. The use of the catheter 500 and pump/filtration system 600 may allow for the IT infusion of AmB and/or other drugs. Unexpectedly, the catheter 500 and pump/filtration system 600 may also be used to reduce, filter (e.g., with the first filter 672, the second filter 682, or both), or otherwise remove some drugs such as AmB. Because of this, the dosage of AmB can be precisely titrated to a desired dose. If levels of AmB reach undesired levels (e.g., undesired high levels), the catheter 500 and pump/filtration system 600 can be used to quickly remove unwanted quantities of AmB from the CSF.

The catheter 500 and pump/filtration system 600 can also be used to deliver a number of other drugs including drugs where the difference between therapeutic doses and toxic doses are relatively small. For example, a drug may be infused into the CSF using the catheter 500 and pump/filtration system 600. If signs of toxicity are observed or if measurements of the drug concentration in the CSF is higher than desired, the catheter 500 and pump/filtration system 600 can be used to rapidly remove the drug from the CSF. Thus, the catheter 500 and pump/filtration system 600 can be used for controlled delivery of drugs into the CSF of patients and the rapid removal of drugs from the CSF, as desired.

The catheter 500 and pump/filtration system 600 may also help to reduce ICP associated with a number of conditions. For example, some conditions (e.g., such as cancer, HSE, and others) may be associated with higher ICP due to cells (e.g., tumor cells, etc.), inflammatory agents, and the like blocking, clogging, or otherwise impacting natural pathways for reabsorption of CSF. By using the catheter 500 and pump/filtration system 600, materials that might blocking natural reabsorption pathways can be removed/reduced, thereby desirably impacting the volume of CSF and reducing ICP.

Systems are also contemplated that utilize a first port for providing access to the cerebrospinal space and/or the CNS at a first location and a second port for providing access the cerebrospinal space and/or the CNS at a second location. Such ports may be implanted acutely or for extended periods of time. In some instances, the ports may allow for infusion of substances to the cerebrospinal space and/or the CNS, removal of substances from the cerebrospinal space and/or the CNS, or both. One or both of the ports may be or otherwise be similar to an Ommaya reservoir. The ports may be designed to be used with a tube/catheter, the catheter 500, and/or the pump/filtration system 600. For example, a first tube and/or first catheter 500 may be connected with or otherwise be connectable to one of the ports and a second tube and/or second catheter 500 may be connected with or otherwise be connectable to the other port. CSF may be removed from the patient (e.g., using a tube, either the first or the second catheter 500, or the like) and filtered by the pump/filtration system 600. In some instances, the filtered CSF may be returned to the patient using the same tube/catheter. In other instances, the filtered CSF may be returned to the patient using the other tube/catheter. In other words, CSF may be removed from the patient using a catheter at the first port, filtered, and then returned to the patient using a catheter at the second port. This may form a loop-like pathway the helps to circulate CSF through the cerebrospinal space and/or the CNS. The ports may be positioned along the patient in a manner that helps to facilitate circulation of CSF. For example, one of the ports may be positioned at the cranium of the patient (e.g., which may include providing access to the ventricles of the brain) and the other may be positioned along a lumbar region of the spine (e.g., which may provide access to the cerebrospinal space at a position adjacent to the lumbar space). Other locations are contemplated.

All directional references (e.g., proximal, distal, upper, lower, upward, downward, left, right, lateral, front, back, top, bottom, above, below, vertical, horizontal, clockwise, and counterclockwise) are only used for identification purposes to aid the reader's understanding of the present disclosure, and do not create limitations, particularly as to the position, orientation, or use of the disclosure. Connection references (e.g., attached, coupled, connected, and joined) are to be construed broadly and may include intermediate members between a collection of elements and relative movement between elements unless otherwise indicated. As such, connection references do not necessarily infer that two elements are directly connected and in fixed relation to each other. It should be noted that delivery sheath and delivery catheter may be used interchangeably for purposes of this description. The exemplary drawings are for purposes of illustration only and the dimensions, positions, order and relative sizes reflected in the drawings attached hereto may vary.

The above specification, examples and data provide a complete description of the structure and use of exemplary embodiments of the disclosure as claimed below. Although various embodiments of the disclosure as claimed have been described above with a certain degree of particularity, or with reference to one or more individual embodiments, those skilled in the art could make numerous alterations to the disclosed embodiments without departing from the scope of this disclosure. Other embodiments are therefore contemplated. It is intended that all matter contained in the above description and shown in the accompanying drawings shall be interpreted as illustrative only of particular embodiments and not limiting. Changes in detail or structure may be made without departing from the basic elements of the disclosure.

## Claims

1. A system for removing materials from cerebrospinal fluid, the system comprising:
a first port for providing access to a cerebrospinal space at a first location;
a first catheter (500) coupled to the first port, the first catheter being designed to remove cerebrospinal fluid from the first location;
a filtration system (600) coupled to the first catheter (500), the filtration system being designed to filter the cerebrospinal fluid removed from the first location, wherein the filtration system includes a filter component configured to remove one or more gram-negative meningitis causing bacterial pathogens selected from Pseudomonas bacteria, Acinetobacter bacteria, Klebsiella bacteria, or combinations thereof, and one or more cytokines selected from IL-1ra, IL-6, TNF, CRP, CXCL10, or combinations thereof, wherein the filtration system (600) includes a first filter (672) to separate cerebrospinal fluid into a first clean cerebrospinal pathway (678) and a first waste pathway (680), a second filter (682) configured to separate the first waste pathway (680) into a second clean cerebrospinal pathway (686) and a second waste pathway (690), a waste collection apparatus (696) in fluid communication with the second waste pathway (690), and a return outlet (698) in fluid communication with the first clean cerebrospinal pathway (678) and the second clean cerebrospinal pathway (686);
a second port for providing access to the cerebrospinal space at a second location; and
a second catheter coupled to the second port, the second catheter being designed to return the filtered cerebrospinal fluid.

2. The system of claim 1, wherein the first location is a lumbar region of a patient.

3. The system of claim 1 or 2, wherein the second location is adjacent to a cranial region of the patient.

4. The system of any one of claims 1-3, wherein the filter component is further configured to remove one or more endotoxins from the cerebrospinal fluid.

5. The system of any one of claims 1-4, wherein the filter component comprises a tangential flow filtration (TFF) filter.

6. The system of any one of claims 1-5, wherein the filtration system includes a 5 kDa tangential flow filter, a 100 kDa tangential flow filter, or both.

7. The system of any one of claims 1-6, wherein the filter component is capable of removing material having a mass in the range of 1 to 500 kDa.

8. The system of any one of claims 1-7, wherein the filter component has a pore size in the range of 0.1 to 5 microns.

9. The system of any one of claims 1-8, wherein at least one of the first catheter (500) or the second catheter includes a reinforcing coil.

10. The system of claim 9, wherein at least one of the first catheter (500) or the second catheter includes a plurality of openings (532A, 532B, 532A) disposed between windings of the reinforcing coil.

## Patentansprüche

1. System zum Entfernen von Materialien aus Cerebrospinalflüssigkeit, wobei das System aufweist:
einen ersten Anschluss zum Bereitstellen eines Zugangs zu einem Cerebrospinalraum an einer ersten Stelle;
einen mit dem ersten Anschluss verbundenen ersten Katheter (500), wobei der erste Katheter dafür konfiguriert ist, Cerebrospinalflüssigkeit von der ersten Stelle zu entfernen;
ein mit dem ersten Katheter (500) verbundenes Filtersystem (600), wobei das Filtersystem dafür konfiguriert ist, die von der ersten Stelle entnommene Cerebrospinalflüssigkeit zu filtern, wobei das Filtersystem eine Filterkomponente aufweist, die dafür konfiguriert ist, einen oder mehrere gramnegative, Meningitis verursachende bakterielle Krankheitserreger, ausgewählt aus Pseudomonas-Bakterien, Acinetobacter-Bakterien, Klebsiella-Bakterien oder Kombinationen davon, und ein oder mehrere Zytokine, ausgewählt aus IL-1ra, IL-6, TNF, CRP, CXCL10 oder Kombinationen davon, zu entfernen, wobei das Filtersystem (600) aufweist: einen ersten Filter (672) zum Trennen von Cerebrospinalflüssigkeit in einen ersten reinen Cerebrospinalpfad (678) und einen ersten Abfallproduktpfad (680), einen zweiten Filter (682), der dafür konfiguriert ist, den ersten Abfallproduktpfad (680) in einen zweiten reinen Cerebrospinalpfad (686) und einen zweiten Abfallproduktpfad (690) zu trennen, eine Abfallproduktsammelvorrichtung (696), die in Fluidkommunikation mit dem zweiten Abfallproduktpfad (690) steht, und einen Rücklaufauslass (698), der in Fluidkommunikation mit dem ersten reinen Cerebrospinalpfad (678) und dem zweiten reinen Cerebrospinalpfad (686) steht;
einen zweiten Anschluss zum Bereitstellen eines Zugangs zum Cerebrospinalraum an einer zweiten Stelle; und
einen mit dem zweiten Anschluss verbundenen zweiten Katheter, wobei der zweite Katheter dafür konfiguriert ist, die gefilterte Cerebrospinalflüssigkeit zurückzuführen.

2. System nach Anspruch 1, wobei die erste Stelle ein Lendenbereich eines Patienten ist.

3. System nach Anspruch 1 oder 2, wobei die zweite Stelle einem kranialen Bereich des Patienten benachbart ist.

4. System nach einem der Ansprüche 1 bis 3, wobei die Filterkomponente ferner dafür konfiguriert ist, ein oder mehrere Endotoxine aus der Cerebrospinalflüssigkeit zu entfernen.

5. System nach einem der Ansprüche 1 bis 4, wobei die Filterkomponente einen Tangentialstromfilter (TFF) aufweist.

6. System nach einem der Ansprüche 1 bis 5, wobei das Filtersystem einen 5-kDa-Tangentialstromfilter und/oder einen 100-kDa-Tangentialstromfilter aufweist.

7. System nach einem der Ansprüche 1 bis 6, wobei die Filterkomponente in der Lage ist, Materialien mit einer Masse im Bereich von 1 bis 500 kDa zu entfernen.

8. System nach einem der Ansprüche 1 bis 7, wobei die Filterkomponente eine Porengröße im Bereich von 0,1 bis 5 Mikrometer aufweist.

9. System nach einem der Ansprüche 1 bis 8, wobei der erste Katheter (500) und/oder der zweite Katheter eine Verstärkungsspirale aufweist.

10. System nach Anspruch 9, wobei der erste Katheter (500) und/oder der zweite Katheter eine Vielzahl von Öffnungen (532A, 532B, 532A) aufweist, die zwischen den Windungen der Verstärkungsspirale angeordnet sind.

## Revendications

1. Système pour éliminer des matières dans du liquide céphalo-rachidien, le système comprenant :
un premier orifice pour permettre l'accès à l'espace céphalo-rachidien en un premier emplacement ;
un premier cathéter (500) couplé au premier orifice, le premier cathéter étant conçu pour soutirer du liquide céphalo-rachidien depuis le premier emplacement ;
un système de filtration (600) couplé au premier cathéter (500), le système de filtration étant conçu pour filtrer le liquide céphalo-rachidien soutiré depuis le premier emplacement, lequel système de filtration comprend un composant de filtre configuré pour éliminer un ou plusieurs pathogènes bactériens causant une méningite Gram négatifs choisis parmi les bactéries Pseudomonas, les bactéries Acinetobacter, les bactéries Klebsiella, ou des combinaisons de celles-ci, et une ou plusieurs cytokines choisies parmi IL-1ra, IL-6, TNF, CRP, CXCL10, ou des combinaisons de celles-ci, lequel système de filtration (600) comprend un premier filtre (672) pour séparer le liquide céphalo-rachidien en une première voie céphalo-rachidienne propre (678) et une première voie de déchet (680), un deuxième filtre (682) configuré pour séparer la première voie de déchet (680) en une deuxième voie céphalo-rachidienne propre (686) et une deuxième voie de déchet (690), un appareil de collecte de déchet (696) en communication de fluide avec la deuxième voie de déchet (690), et une sortie de retour (698) en communication de fluide avec la première voie céphalo-rachidienne propre (678) et la deuxième voie céphalo-rachidienne propre (686) ;
un deuxième orifice pour permettre l'accès à l'espace céphalo-rachidien en un deuxième emplacement ; et
un deuxième cathéter couplé au deuxième orifice, le deuxième cathéter étant conçu pour renvoyer le liquide céphalo-rachidien filtré.

2. Système selon la revendication 1, dans lequel le premier emplacement est une région lombaire du patient.

3. Système selon la revendication 1 ou 2, dans lequel le deuxième emplacement est adjacent à une région crânienne du patient.

4. Système selon l'une quelconque des revendications 1 à 3, dans lequel le composant de filtre est en outre configuré pour éliminer une ou plusieurs endotoxines du liquide céphalo-rachidien.

5. Système selon l'une quelconque des revendications 1 à 4, dans lequel le composant de filtre comprend un filtre à filtration par écoulement tangentiel (TFF).

6. Système selon l'une quelconque des revendications 1 à 5, dans lequel le système de filtration comprend un filtre à écoulement tangentiel de 5 kDa, un filtre à écoulement tangentiel de 100 kDa, ou les deux.

7. Système selon l'une quelconque des revendications 1 à 6, dans lequel le composant de filtre est capable d'éliminer des matières ayant une masse située dans la plage allant de 1 à 500 kDa.

8. Système selon l'une quelconque des revendications 1 à 7, dans lequel le composant de filtre a une taille de pores située dans la plage allant de 0,1 à 5 micromètres.

9. Système selon l'une quelconque des revendications 1 à 8, dans lequel au moins l'un parmi le premier cathéter (500) et le deuxième cathéter comprend une bobine de renforcement.

10. Système selon la revendication 9, dans lequel au moins l'un parmi le premier cathéter (500) et le deuxième cathéter comprend une pluralité d'ouvertures (532A, 532B, 532A) disposées entre des enroulements de la bobine de renforcement.
